Europäisches Patentamt

European Patent Office

Office européen des brevets

(19)

(11) **EP 0 700 894 B1**

(12) **FASCICULE DE BREVET EUROPEEN**

(45) Date de publication et mention
de la délivrance du brevet:
**07.04.1999 Bulletin 1999/14**

(51) Int Cl.$^6$: **C07C 205/24**

(21) Numéro de dépôt: **95402032.7**

(22) Date de dépôt: **08.09.1995**

(54) **Procédé de préparation de l'acide picrique**

Verfahren zur Herstellung von Pikrinsaüre

Process for the preparation of picric acid

(84) Etats contractants désignés:
**BE DE FR GB IT**

(30) Priorité: **09.09.1994 FR 9410781**

(43) Date de publication de la demande:
**13.03.1996 Bulletin 1996/11**

(73) Titulaire: **RHODIA CHIMIE**
**92408 Courbevoie Cédex (FR)**

(72) Inventeurs:
• **Metivier, Pascal**
**F-69110 Sainte Foy Les Lyon (FR)**

• **Bernard, Laurent**
**F-69200 Venissieux (FR)**

(74) Mandataire: **Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
GB-A- Q11 005        US-A- 1 349 802
US-A- 1 393 714

**Description**

La présente invention a pour objet un procédé de préparation de l'acide picrique.

L'acide picrique ou 2,4,6-trinitrophénol est un produit couramment utilisé dans l'industrie et plus particulièrement dans la fabrication d'explosifs, de médicaments, de colorants et autres applications. [Tadeusz Urbanski, Chemistry and Technology of Explosives, Pergamon Press, pp. 499 (1964)]

Il existe deux grandes voies d'accès à l'acide picrique, décrites dans la littérature à savoir la sulfonitration du phénol ou la nitration du dinitrophénol préparé par hydrolyse du chlorodinitrobenzène.

La première consiste à conduire le procédé en deux étapes comprenant une étape de sulfonation du phénol puis une étape de nitration du phénol sulfoné obtenu.

Toutefois, un tel procédé souffre de plusieurs inconvénients : la sulfonation est longue et peu productive et la nitration se fait alors en milieu déjà dilué ce qui conduit à une chute de la productivité.

L'autre mode de préparation de l'acide picrique comprend également plusieurs étapes : nitration du monochlorobenzène en chlorodinitrobenzène suivie d'une hydrolyse du produit obtenu puis nitration du dinitrophénol par un mélange d'acide nitrique et d'oléum.

Ledit procédé ne donne également pas satisfaction car il est long, complexe et polluant en raison de la formation de chlorure de sodium à l'étape d'hydrolyse. Il se pose alors le problème du traitement des effluents aqueux salins.

L'objet de la présente invention est de fournir un procédé permettant d'obvier aux inconvénients précités.

Il a maintenant été trouvé et c'est ce qui constitue l'objet de la présente invention un procédé de préparation de l'acide picrique caractérisé par le fait qu'il consiste à effectuer la nitration de l'o-nitrophénol et/ou du p-nitrophénol en dinitrophénol(s) de telle sorte que le ou lesdits produit(s) intermédiaire(s) formé(s) reste(nt) solubles dans le milieu réactionnel, et à poursuivre la nitration du ou des dinitrophénols afin d'obtenir l'acide picrique qui précipite.

Dans le procédé de l'invention, on forme un dinitrophénol comme produit intermédiaire. Il s'agit plus précisément, du 2,4-dinitrophénol lorsque le substrat de départ est le p-nitrophénol et d'un mélange d'isomères, à savoir le 2,4-dinitrophénol et le 2,6-dinitrophénol obtenus à partir de l'o-nitrophénol.

Il a été découvert de manière inattendue qu'il était possible de préparer l'acide picrique à partir de l'o-nitrophénol et/ou du p-nitrophénol dans la mesure où l'intermédiaire formé ne précipite pas dans la milieu car il s'avère qu'une fois précipité, il n'est plus possible de le nitrer.

Il importe donc de contrôler l'absence de précipitation du 2,4-dinitrophénol ou du mélange de 2,4- et de 2,6-dinitrophénols.

Conformément à l'invention, le ou lesdits produit(s) intermédiaire(s) formé(s) doivent rester solubles dans le milieu réactionnel ce qui implique un contrôle de la concentration du substrat de départ qui dépend du mode de mise en oeuvre, selon qu'il soit en discontinu ou en continu. Il est évidemment possible de tolérer une faible quantité de produits intermédiaires sous forme précipitée, de préférence, moins de 3 %, et encore plus préférentiellement moins de 1 %, mais ceci au détriment du rendement réactionnel.

Un première mode de réalisation de l'invention consiste à introduire l'acide nitrique ou un mélange nitrant dans l'o-nitrophénol et/ou dans le p-nitrophénol.

Par "mélange nitrant", on entend l'acide nitrique associé comme précisé ci-après à un co-acide fort.

L'o-nitrophénol peut être mis en oeuvre, sous forme solide, à l'état fondu ou en solution dans le co-acide. Quant au p-nitrophénol, il peut être mis en oeuvre sous forme solide, en solution dans le co-acide ou en mélange avec l'eau, sous forme d'eutectique.

Dans ce mode de réalisation en particulier, il y a lieu de veiller à ce que la concentration du ou des dinitrophénols dans le milieu réactionnel soit telle qu'il(s) reste(nt) soluble(s) dans les conditions réactionnelles.

Ainsi, à une température donnée, la concentration de dinitrophénol(s) devra être maintenue inférieure à la concentration où commence la précipitation du ou des dinitrophénols.

La quantité de dinitrophénol(s) solubilisé(s) dépend de la température réactionnelle et de l'acidité du milieu.

On précisera, à titre indicatif et sans caractère limitatif, que la solubilité du 2,4-dinitrophénol à 65°C, dans une solution d'acide sulfurique à 95 %, est de 12 %.

Il est à noter que le contrôle de la concentration en produit(s) intermédiaire(s) s'applique également aux autres modes d'introduction des réactifs. Toutefois, la concentration étant un facteur limitant la productivité, d'autres modes de contrôle qui sont préférés, peuvent être appliqués.

Une autre variante d'exécution de la présente invention consiste à introduire progressivement le p-nitrophénol et/ou l'o-nitrophénol dans le milieu réactionnel comprenant l'acide nitrique ou le mélange nitrant ou à introduire simultanément en parallèle le p-nitrophénol et/ou l'o-nitrophénol et l'acide nitrique ou le mélange nitrant sur un pied cuve initial comprenant de l'eau ou le co-acide.

Par introduction progressive, on entend une addition en continu ou en plusieurs fractions.

L'o-nitrophénol peut être mis en oeuvre, sous forme solide, à l'état fondu ou en solution dans le co-acide.

Quant au p-nitrophénol, il peut être mis en oeuvre sous forme solide, en solution dans le co-acide ou en mélange avec l'eau, sous forme d'eutectique.

Dans pareil cas, la vitesse d'introduction de l'o-nitrophénol et/ou du p-nitrophénol doit être choisie inférieure à la vitesse de nitration de ou des dinitrophénols.

La vitesse de nitration dépend de nombreux paramètres, notamment de la température, de la concentration en acide nitrique, co-acide et dinitrophénol(s).

Dans ce cas, il est possible d'avoir une concentration en substrat de départ plus élevée tout en maintenant la solubilité du ou des dinitrophénols dans le milieu.

On peut choisir la bonne vitesse de nitration, en effectuant des dosages de routine, en analysant le précipité obtenu qui doit être de l'acide picrique.

Les produits de départ intervenant dans le procédé de l'invention sont l'o-nitrophénol ou le p-nitrophénol ou leurs mélanges.

L'Homme de métier peut en fonction de la température et de l'acidité du milieu déterminer la concentration des sustrats de départ de telle sorte que les conditions précisées en matière de solubilité des produits intermédiaires soient respectées et ceci quel que soit le mode de mise en oeuvre en discontinu ou en continu.

Lorsque le substrat de départ est introduit dans la solution de co-acide, sa concentration est avantageusement comprise entre 20 % et 80 %, de préférence, entre 25 % et 35 % en poids.

Conformément au procédé de l'invention, l'opération de nitration peut être conduite selon deux modes de réalisation.

Une première variante consiste à ne mettre en oeuvre que l'acide nitrique.

On fait appel à une solution aqueuse d'acide nitrique ayant une concentration indifférente pouvant varier entre 30 % et 100 %. Toutefois, une concentration comprise entre 68 % et 100 % est préférée.

Dans pareil cas, l'acide nitrique est mis en jeu, en large excès. La quantité d'acide nitrique représente de 4 à 10 fois le poids d'o-nitrophénol et/ou de p-nitrophénol.

Un autre variante de l'invention consiste à associer l'acide nitrique à un autre acide fort dénommé ensuite de manière simplifiée "co-acide".

Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation nitrique.

On peut citer plus particulièrement les oxyacides halogénés ou non tels que l'acide sulfurique, l'acide pyrosulfurique, l'acide phosphorique, les acides polyphosphoriques, l'acide perchlorique, les acides halogénosulfoniques tels que l'acide fluorosulfonique, l'acide chlorosulfonique ou l'acide trifluorométhanesulfonique, l'acide méthanesulfonique, l'acide éthane-sulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on choisit, tout particulièrement, l'acide sulfurique ou l'acide phosphorique.

Un co-acide préféré est l'acide sulfurique et l'on fait appel de préférence à une solution concentrée d'acide sulfurique : la concentration en acide sulfurique est choisie de préférence supérieure à 90 % en poids et encore plus préférentiellement supérieure à 98 % en poids.

Il est possible de substituer l'acide sulfurique par les oléums : ceux-ci comprenant du $SO_3$ en quantités variables, de préférence de 20 à 50 %.

La quantité d'acide nitrique mise en oeuvre exprimée par le rapport du nombre d'acide nitrique et le nombre de moles d'o-nitrophénol et/ou p-nitrophénol peut varier entre environ 2 et environ 3, et plus particulièrement entre 2 et 2,2.

La quantité de co-acide utilisé peut varier dans de larges limites. Elle représente de 1 à 10 fois le poids d'o-nitrophénol et/ou de p-nitrophénol.

Le procédé de l'invention est avantageusement conduit à une température comprise entre 40 et 100°C, de préférence entre 40°C et 70°C.

Le procédé de l'invention est généralement mis en oeuvre sous pression atmosphérique mais peut l'être également sous pression légèrement réduite comprise, par exemple, entre 500 et 760 mm de mercure.

Il n'est pas nécessaire d'établir une atmosphère de gaz rares.

D'un point de vue pratique, le procédé de l'invention est facile à mettre en oeuvre car il ne nécessite pas d'avoir recours à un appareillage spécifique.

Pratiquement, le procédé de l'invention peut être mis en oeuvre de la manière décrite ci-après.

On charge les différents constituants du mélange réactionnel dans l'appareillage choisi.

Plusieurs modes de mises en oeuvre qui ont déjà été précisés, peuvent être envisagés et que l'on peut résumer ainsi :

- introduction de l'acide nitrique ou du mélange nitrant dans l'o-nitrophénol et/ou p-nitrophénol,
- introduction de l'o-nitrophénol et/ou p-nitrophénol dans l'acide nitrique ou le mélange nitrant,
- introduction en parallèle de l'o-nitrophénol et/ou p-nitrophénol et de l'acide nitrique ou du mélange nitrant sur un pied de cuve comprenant de l'eau ou le co-acide.

La dernière variante est le mode préféré : le pied de cuve étant constitué par une solution de co-acide, de préférence une solution d'acide sulfurique concentré, et ayant plus préférentiellement une concentration supérieure à 90 %.

La durée d'addition de l'o-nitrophénol et/ou du p-nitrophénol peut varier, par exemple, entre 30 minutes et 4 heures, de préférence entre 1 et 2 heures.

Après mise en oeuvre des réactifs, on maintient le mélange réactionnel dans la zone de température précitée pendant quelques instants (15 à 30 minutes).

En fin de réaction, l'acide picrique précipite dans le milieu.

Il peut être séparé selon les techniques classiques de séparation solide/liquide, de préférence par filtration.

On lave une ou plusieurs fois, le produit obtenu, de préférence 2 fois, avec l'eau.

On récupère ainsi l'acide picrique.

Un avantage du procédé de l'invention est que l'acide picrique est produit sans passage par une phase fondue ce qui permet d'éviter certains dangers d'explosions au niveau de la fabrication.

Les exemples qui suivent, illustrent l'invention sans toutefois la limiter.

Dans les exemples, les rendements mentionnés correspondent à la définition suivante :

$$\text{Rendement : RR} = \frac{\text{nombre de moles d'acide picrique formées}}{\text{nombre de moles d'o-nitrophénol (ou de p-nitrophénol) introduites}} \%$$

$$\text{Rendement : RT} = \frac{\text{nombre de moles d'acide picrique formées}}{\text{nombre de moles d'o-nitrophénol (ou p-nitrophénol) transformées}} \%$$

Les abréviations utilisées dans les exemples, ont la signification suivante :

- ONP = o-nitrophénol
- PNP = p-nitrophénol

Exemple 1

Dans un tube shott équipé d'un dispositif d'agitation, d'un réfrigérant, d'un compte-bulles et de deux pousse-seringues et chauffé par bain thermostaté, on charge :

- 15 g d'une solution aqueuse d'acide sulfurique à 95 %,
- 2,74 g (20 mmol) d'o-nitrophénol.

On additionne sous agitation, 9,58 g (soit 60,8 mmol d'acide nitrique) d'un mélange sulfonitrique contenant 57 % d'acide sulfurique, 40 % d'acide nitrique et 3 % d'eau.

On commence par ajouter le tiers dudit mélange, à 43°C, en 24 minutes puis, les deux tiers, à 65°C en 45 minutes. On maintient ensuite le chauffage à 65°C, pendant 15 minutes.

On refroidit le milieu réactionnel, et on le verse sur un mélange de 300 ml eau-glace/acide sulfamique (1 g) et une quantité suffisante de méthanol pour obtenir un volume total de 1 000 ml.

Les réactifs n'ayant pas réagi et les produits obtenus sont dosés par chromatographie liquide haute performance.

Les résultats obtenus sont consignés dans le tableau (I):

Tableau (I)

| Ref. ex. | Substrat | Rapport molaire $HNO_3$/ONP | Ordre des charges | | Température (°C) | Durée (mn) | RR-RT acide picrique |
|---|---|---|---|---|---|---|---|
| | | | En pied | Coulée | | | |
| 1 | ONP | 3 | ONP | mélange sulfonitrique | 43 | 24 | 94 % |
| | | | | | 65 | 45 + 15 | |

Exemple 2

Dans un tricol de 50 ml équipé d'un dispositif d'agitation, d'un réfrigérant, d'un compte-bulles et d'un pousse-seringue et chauffé par bain thermostaté, on charge 20 g d'un mélange sulfonitrique contenant 57 % d'acide sulfurique, 39 % d'acide nitrique et 4 % d'eau (soit 124 mmol d'acide nitrique).

On chauffe sous agitation jusqu'à 67°C puis l'on coule en 42 minutes, 23,76 g (34,2 mmol) d'o-nitrophénol en solution à 20 % dans l'acide sulfurique à 95 %.

On maintient ensuite le chauffage à 67°C, pendant 15 minutes.

On refroidit le milieu réactionnel, et on le verse sur un mélange de 300 ml eau-glace/acide sulfamique (1 g) et une quantité suffisante de méthanol pour obtenir un volume total de 1 000 ml.

Les réactifs n'ayant pas réagi et les produits obtenus sont dosés par chromatographie liquide haute performance.

Les résultats obtenus sont consignés dans le tableau (II) :

Tableau (II)

| Ref. ex. | Substrat | Rapport molaire $HNO_3$/ONP | Ordre des charges | | Température (°C) | Durée (mn) | RR-RT acide picrique |
|---|---|---|---|---|---|---|---|
| | | | En pied | Coulée | | | |
| 2 | ONP | 3,6 | mélange sulfonitrique | ONP | 67 | 42 + 15 | 84 % |

Exemple 3

Dans un tricol de 100 ml équipé d'un dispositif d'agitation, d'un réfrigérant, d'un compte-bulles et de deux pousse-seringues et chauffé par bain thermostaté, on charge 18,3 g d'une solution aqueuse d'acide sulfurique à 95 %.

On chauffe sous agitation jusqu'à 67°C puis l'on coule, en 60 minutes, simultanément 28,2 g (40,6 mmol) d'o-nitrophénol en solution à 20 % dans l'acide sulfurique à 95 % et 19,52 g d'un mélange sulfonitrique contenant 57 % d'acide sulfurique, 39 % d'acide nitrique et 4 % d'eau (soit 121 mmol d'acide nitrique).

On maintient ensuite le chauffage à 67°C, pendant 15 minutes.

On refroidit le milieu réactionnel, et on le verse sur un mélange de 300 ml eau-glace/acide sulfamique (1 g) et une quantité suffisante de méthanol pour obtenir un volume total de 1 000 ml.

Les réactifs n'ayant pas réagi et les produits obtenus sont dosés par chromatographie liquide haute performance.

Les résultats obtenus sont consignés dans le tableau (III) :

Tableau (III)

| Ref. ex. | Substrat | Rapport molaire HNO$_3$/ONP | Ordre des charges | | Température (°C) | Durée (mn) | RR-RT acide picrique |
|---|---|---|---|---|---|---|---|
| | | | En pied | Coulée | | | |
| 3 | ONP | 3 | H$_2$SO$_4$ 95 % | simultanée ONP mélange sulfonitrique | 67 | 60 + 15 | 95 % |

Exemple 4

Dans un tricol de 100 ml équipé d'un dispositif d'agitation, d'un réfrigérant, d'un compte-bulles et de deux pousse-seringues et chauffé par bain thermostaté, on charge 18,3 g d'une solution aqueuse d'acide sulfurique à 95 %.
On chauffe sous agitation jusqu'à 67°C puis l'on coule, en 56 minutes, simultanément 28,34 g (40,8 mmol) d'o-nitrophénol en solution à 20 % dans l'acide sulfurique à 95 % et 13,58 g d'un mélange sulfonitrique contenant 57 % d'acide sulfurique, 39 % d'acide nitrique et 4 % d'eau (soit 84 mmol d'acide nitrique).
On maintient ensuite le chauffage à 67°C, pendant 19 minutes.
On refroidit le milieu réactionnel, et on le verse sur un mélange de 300 ml eau-glace/acide sulfamique (1 g) et une quantité suffisante de méthanol pour obtenir un volume total de 1 000 ml.
Les réactifs n'ayant pas réagi et les produits obtenus sont dosés par chromatographie liquide haute performance.
Les résultats obtenus sont consignés dans le tableau (IV) :

Tableau (IV)

| Ref. ex. | Substrat | Rapport molaire HNO$_3$/ONP | Ordre des charges | | Température (°C) | Durée (mn) | RR-RT acide picrique |
|---|---|---|---|---|---|---|---|
| | | | En pied | Coulée | | | |
| 4 | ONP | 2 | H$_2$SO$_4$ 95 % | simultanée ONP mélange sulfonitrique | 67 | 56 + 19 | 88 % |

Exemple 5

Dans un tricol de 100 ml équipé d'un dispositif d'agitation, d'un réfrigérant, d'un compte-bulles et de deux pousse-seringues et chauffé par bain thermostaté, on charge 18,3 g d'une solution aqueuse d'acide sulfurique à 95 %.
On chauffe sous agitation jusqu'à 67°C puis l'on coule en 60 minutes, simultanément 28,2 g (40,6 mmol) de p-nitrophénol en solution à 20 % dans l'acide sulfurique à 95 % et 19,52 g d'un mélange sulfonitrique contenant 57 % d'acide sulfurique, 39 % d'acide nitrique et 4 % d'eau (soit 121 mmol d'acide nitrique).
On maintient ensuite le chauffage à 67°C, pendant 15 minutes.
On refroidit le milieu réactionnel, et on le verse sur un mélange de 300 ml eau-glace/acide sulfamique (1 g) et une quantité suffisante de méthanol pour obtenir un volume total de 1 000 ml.
Les réactifs n'ayant pas réagi et les produits obtenus sont dosés par chromatographie liquide haute performance.
Les résultats obtenus sont consignés dans le tableau (V) :

Tableau (V)

| Ref. ex. | Substrat | Rapport molaire $HNO_3/PNP$ | Ordre des charges | | Température (°C) | Durée (mn) | RR-RT acide picrique |
|---|---|---|---|---|---|---|---|
| | | | En pied | Coulée | | | |
| 5 | PNP | 3 | $H_2SO_4$ 95 % | simultanée PNP mélange sulfonitrique | 67 | 60 + 15 | 98 % |

**Revendications**

1.  Procédé de préparation de l'acide picrique caractérisé par le fait qu'il consiste à effectuer la nitration de l'o-nitrophénol et/ou du p-nitrophénol en dinitrophénol(s) de telle sorte que le ou lesdits produit(s) intermédiaire(s) formé(s) restent solubles dans le milieu réactionnel, et à poursuivre la nitration du ou des dinitrophénol(s) afin d'obtenir l'acide picrique qui précipite.

2.  Procédé selon la revendication 1 caractérisé par le fait qu'il consiste à introduire l'acide nitrique ou le mélange nitrant dans l'o-nitrophénol et/ou dans le p-nitrophénol.

3.  Procédé selon la revendication 1 caractérisé par le fait qu'il consiste à introduire progressivement le p-nitrophénol et/ou l'o-nitrophénol dans le milieu réactionnel comprenant l'acide nitrique ou le mélange nitrant ou à introduire simultanément en parallèle le p-nitrophénol et/ou l'o-nitrophénol et l'acide nitrique ou le mélange nitrant sur un pied cuve initial comprenant de l'eau ou le co-acide.

4.  Procédé selon l'une des revendications 1 à 3 caractérisé par le fait que l'o-nitrophénol est mis en oeuvre sous forme solide, à l'état fondu ou en solution dans le co-acide et que le p-nitrophénol est mis en oeuvre, sous forme solide, en solution dans le co-acide ou en mélange avec l'eau, sous forme d'eutectique.

5.  Procédé selon l'une des revendications 1 à 4 caractérisé par le fait que la concentration de dinitrophénol(s) est maintenue inférieure à la concentration où commence la précipitation du ou des dinitrophénols.

6.  Procédé selon la revendication 3 caractérisé par le fait que la vitesse d'introduction de l'o-nitrophénol et/ou du p-nitrophénol est choisie inférieure à la vitesse de nitration de ou des dinitrophénols.

7.  Procédé selon l'une des revendications 1 à 6 caractérisé par le fait que la nitration est effectuée à l'aide d'acide nitrique.

8.  Procédé selon l'une des revendications 1 à 6 caractérisé par le fait que la nitration est effectuée à l'aide d'un mélange comprenant l'acide nitrique et un co-acide fort.

9.  Procédé selon la revendication 8 caractérisé par le fait que le co-acide est un oxyacide halogéné ou non ou un acide halogénosulfonique.

10. Procédé selon la revendication 9 caractérisé par le fait que le co-acide est l'acide sulfurique, l'acide phosphorique ou un oléum.

11. Procédé selon l'une des revendications 8 à 10 caractérisé par le fait que la concentration en acide sulfurique est choisie de préférence supérieure à 90 % en poids et encore plus préférentiellement supérieure à 98 % en poids.

12. Procédé selon l'une des revendications 1 à 11 caractérisé par le fait que la concentration de la solution aqueuse d'acide nitrique varie entre 30 % et 100 %, de préférence, entre 68 % et 100 %.

13. Procédé selon la revendication 7 caractérisé par le fait que la quantité d'acide nitrique représente de 4 à 10 fois le poids d'o-nitrophénol et/ou de p-nitrophénol.

**14.** Procédé selon la revendication 8 caractérisé par le fait que la quantité d'acide nitrique mise en oeuvre exprimée par le rapport du nombre d'acide nitrique et le nombre de moles d'o-nitrophénol et/ou p-nitrophénol varie entre 2 et 3, et de préférence entre 2 et 2,2.

**15.** Procédé selon la revendication 8 caractérisé par le fait que la quantité de co-acide représente de 1 à 10 fois le poids d'o-nitrophénol et/ou de p-nitrophénol.

**16.** Procédé selon l'une des revendications 1 à 15 caractérisé par le fait que la température réactionnelle est comprise entre 40 et 100°C, de préférence entre 40°C et 70°C.

**Patentansprüche**

**1.** Verfahren zur Herstellung von Pikrinsäure, dadurch gekennzeichnet, daß man eine Nitrierung von ortho-Nitrophenol und/oder para-Nitrophenol in Dinitrophenol(en) derart durchführt, daß das oder die gebildeten Zwischenprodukte in dem Reaktionsmilieu löslich bleiben, und man mit der Nitrierung des oder der Nitrophenole fortfährt, um die Pikrinsäure zu erhalten, die ausfällt.

**2.** Verfahren nach Anspruch 1, dadurch gekennzeichnet, daß man Salpetersäure oder eine Nitriermischung zu dem ortho-Nitrophenol und/oder para-Nitrophenol hinzugibt.

**3.** Verfahren nach Anspruch 1, dadurch gekennzeichnet, daß man allmählich das para-Nitrophenol und/oder das ortho-Nitrophenol zu dem Reaktionsmilieu zugibt, welches die Salpetersäure oder die Nitriermischung enthält, oder man gleichzeitig parallel das para-Nitrophenol und/oder das ortho-Nitrophenol und die Salpetersäure oder die Nitriermischung zu der Vorlage hinzugibt, welche Wasser und die zusätzliche Säure enthält.

**4.** Verfahren nach einem der Ansprüche 1 bis 3, dadurch gekennzeichnet, daß das ortho-Nitrophenol in fester Form, im geschmolzenen Zustand oder in Lösung in der zusätzlichen Säure hinzugegeben wird und daß das para-Nitrophenol in fester Form, in Lösung in der zusätzlichen Säure oder in Mischung mit Wasser in Form eines Eutektikums hinzugegeben wird.

**5.** Verfahren nach einem der Ansprüche 1 bis 4, dadurch gekennzeichnet, daß die Konzentration an Dinitrophenol (en) unterhalb der Konzentration konstantgehalten wird, bei der die Ausfällung des oder der Nitrophenole beginnt.

**6.** Verfahren nach Anspruch 3, dadurch gekennzeichnet, daß die Zugabegeschwindigkeit des ortho-Nitrophenols und/oder des para-Nitrophenols derart ausgewählt ist, daß sie unterhalb der Nitriergeschwindigkeit des oder der Dinitrophenole liegt.

**7.** Verfahren nach einem der Ansprüche 1 bis 6, dadurch gekennzeichnet, daß die Nitrierung mit Hilfe von Salpetersäure durchgeführt wird.

**8.** Verfahren nach einem der Ansprüche 1 bis 6, dadurch gekennzeichnet, daß die Nitrierung mit Hilfe einer Mischung durchgeführt wird, die die Salpetersäure und eine starke weitere Säure umfaßt.

**9.** Verfahren nach Anspruch 8, dadurch gekennzeichnet, daß die weitere Säure eine gegebenenfalls halogenierte Oxysäure oder eine Halogensulfonsäure ist.

**10.** Verfahren nach Anspruch 9, dadurch gekennzeichnet, daß die weitere Säure Schwefelsäure, Phosphorsäure oder ein Oleum ist.

**11.** Verfahren nach einem der Ansprüche 8 bis 10, dadurch gekennzeichnet, daß die Konzentration an Schwefelsäure vorzugsweise mit mehr als 90 Gew.-% ausgewählt ist, insbesondere mit mehr als 98 Gew.-%.

**12.** Verfahren nach einem der Ansprüche 1 bis 11, dadurch gekennzeichnet, daß die Konzentration der wäßrigen Schwefelsäurelösung zwischen 30 % und 100 %, vorzugsweise zwischen 68 % und 100 %, variiert.

**13.** Verfahren nach Anspruch 7, dadurch gekennzeichnet, daß die Menge an Salpetersäure das 4- bis 10fache des Gewichts des ortho-Nitrophenols und/oder des para-Nitrophenols darstellt.

**14.** Verfahren nach Anspruch 8, dadurch gekennzeichnet, daß die eingesetzte Salpetersäuremenge, berechnet als das Verhältnis der Molanzahl Salpetersäure zur Molanzahl ortho-Nitrophenol und/oder para-Nitrophenol zwischen 2 und 3, vorzugsweise zwischen 2 und 2,2, variiert.

**15.** Verfahren nach Anspruch 8, dadurch gekennzeichnet, daß die Menge an weiterer Säure das 1- bis 10fache des Gewichts des ortho-Nitrophenols und/oder des para-Nitrophenols darstellt.

**16.** Verfahren nach einem der Ansprüche 1 bis 15, dadurch gekennzeichnet, daß die Reaktionstemperatur zwischen 40 °C und 100 °C, vorzugsweise zwischen 40 °C und 70 °C, liegt.

**Claims**

**1.** A process for the preparation of picric acid characterised in that it comprises effecting nitration of o-nitrophenol and/or p-nitrophenol to give dinitrophenol(s) in such a way that said intermediate product or products formed remain soluble in the reaction medium, and continuing nitration of the dinitrophenol or dinitrophenols in order to obtain the picric acid which precipitates.

**2.** A process according to claim 1 characterised in that it comprises introducing the nitric acid or the nitrating mixture into the o-nitrophenol and/or into the p-nitrophenol.

**3.** A process according to claim 1 characterised in that it comprises progressively introducing the p-nitrophenol and/or o-nitrophenol into the reaction medium comprising nitric acid or the nitrating mixture or simultaneously introducing in parallel the p-nitrophenol and/or the o-nitrophenol and nitric acid or the nitrating mixture on to an initial bottoms material comprising water or the co-acid.

**4.** A process according to one of claims 1 to 3 characterised in that the o-nitrophenol is used in solid form, in the molten state or in solution in the co-acid and the p-nitrophenol is used in solid form, in solution in the co-acid or in a mixture with water, in the form of an eutectic mixture.

**5.** A process according to one of claim 1 to 4 characterised in that the concentration of dinitrophenol(s) is kept lower than the concentration at which precipitation of the dinitrophenol or dinitrophenols begins.

**6.** A process according to claim 3 characterised in that the rate of introduction of the o-nitrophenol and/or p-nitrophenol is selected to be lower than the rate of nitration of the dinitrophenol or dinitrophenols.

**7.** A process according to one of claim 1 to 6 characterised in that the nitration operation is effected by means of nitric acid.

**8.** A process according to one of claims 1 to 6 characterised in that the nitration operation is effected by means of a mixture comprising nitric acid and a strong co-acid.

**9.** A process according to claim 8 characterised in that the co-acid is a halogenated or non-halogenated oxyacid or a halogenosulphonic acid.

**10.** A process according to claim 9 characterised in that the co-acid is sulphuric acid, phosphoric acid or an oleum.

**11.** A process according to one of claims 8 to 10 characterised in that the concentration of sulphuric acid is selected preferably at higher than 90% by weight and still more preferably higher than 98% by weight.

**12.** A process according to one of claims 1 to 11 characterised in that the concentration of the aqueous solution of nitric acid varies between 30% and 100%, preferably between 68% and 100%.

**13.** A process according to claim 7 characterised in that the amount of nitric acid represents from 4 to 10 times the weight of o-nitrophenol and/or p-nitrophenol.

**14.** A process according to claim 8 characterised in that the amount of nitric acid used expressed by the ratio of the number of nitric acid and the number of moles of o-nitrophenol and/or p-nitrophenol varies between 2 and 3 and preferably between 2 and 2.2.

15. A process according to claim 8 characterised in that the amount of co-acid represents from 1 to 10 times the weight of o-nitrophenol and/or p-nitrophenol.

16. A process according to any one of claim 1 to 15 characterised in that the reaction temperature is between 40 and 100°C, preferably between 40°C and 70°C.